(19) Europäisches Patentamt
European Patent Office
Office européen des brevets

(11) Veröffentlichungsnummer: **0 326 673 A2**

(12) # EUROPÄISCHE PATENTANMELDUNG

(21) Anmeldenummer: 88120104.0

(51) Int. Cl.⁴: **C07H 15/04 , //C07H7/033**

(22) Anmeldetag: 02.12.88

(30) Priorität: 05.02.88 DE 3803465

(43) Veröffentlichungstag der Anmeldung: 09.08.89 Patentblatt 89/32

(84) Benannte Vertragsstaaten: DE FR GB IT

(71) Anmelder: **HÜLS AKTIENGESELLSCHAFT**
**Patentabteilung / PB 15 - Postfach 13 20**
**D-4370 Marl 1(DE)**

(72) Erfinder: **Ripke, Norbert, Dr.**
**Hellweg 28**
**D-4358 Haltern(DE)**
Erfinder: **Thiem, Joachim, Prof. Dr.**
**Asbeckweg 31**
**D-4400 Münster(DE)**
Erfinder: **Böcker, Thomas**
**Kettelerstrasse 75**
**D-4400 Münster(DE)**

(54) **Verfahren zur Herstellung von oxidierten substituierten Sacchariden.**

(57) Alkylglucoside mit kurzen Alkylgruppen können in wäßrigen Lösungen zu Alkylglucuronsäuren oxidiert werden.

Erfindungsgemäß gelingt es nun, auch Saccharide mit langkettigen Alkyl- oder Acylgruppen, die in Wasser begrenzt oder nicht löslich sind, in wäßrigen Mischungen durch Sauerstoff an Platin-Katalysatoren in guten Ausbeuten zu oxidieren.

Herstellung von oxidierten substituierten Sacchariden.

EP 0 326 673 A2

Xerox Copy Centre

# Verfahren zur Herstellung von oxidierten substituierten Sacchariden

Die vorliegende Erfindung betrifft ein Verfahren zur Herstellung von oxidierten substituierten Sacchariden.

Die Oxidation von Glucose zu Glucuronsäure mit Luft an Platin/Aktivkohle-Katalysatoren in wäßriger Lösung ist nach US-PS 2 472 168 bekannt.

Ein demgegenüber verbessertes Verfahren wird in DE-PS 886 305 beschrieben. Dort werden Aldosen zunächst mit kurzkettigen Alkoholen zu wasserlöslichen Alkylglycosiden umgesetzt und dann erst in homogener wäßriger Lösung mit Luft an einem Platin- oder Palladium-Katalysator oxidiert. Nach Spaltung der Glycoside werden Alduronsäuren erhalten.

Nach dieser Patentschrift sind wasserlösliche alkylierte Monosaccharide mit kurzkettigen Alkylgruppen als Zwischenprodukte bekannt. Der Patentschrift ist aber nicht zu entnehmen, wie in Wasser unlösliche oder nur teilweise lösliche Saccharide mit langkettigen Alkyl- oder Acylgruppen in guten Ausbeuten oxidiert werden können.

Aufgabe der vorliegenden Erfindung ist es, ein Verfahren zu entwickeln, nach dem auch oxidierte substituierte Saccharide mit langkettigen Alkyl- und Acylgruppen und mindestens einer Saccharideinheit der Struktur

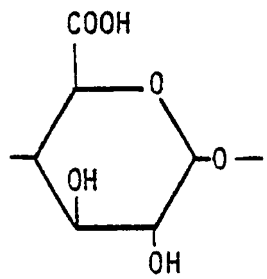

in guten Ausbeuten hergestellt werden können.

Die Aufgabe wird erfindungsgemäß dadurch gelöst, daß man Saccharide mit einem Alkylrest mit 8 bis 22 C-Atomen, der bis zu 2 Hydroxylgruppen enthalten kann, oder mit einem aliphatischen Acylrest mit 8 bis 22 C-Atomen in einer wäßrigen Mischung durch Sauerstoff oder sauerstoffhaltige Gase an einem Katalysator der VIII. Nebengruppe des Periodensystems oxidiert.

Die nach dem erfindungsgemäßen Verfahren hergestellten Saccharide weisen bis zu 60 Saccharideinheiten auf. Außer der genannten Saccharideinheit können sie auch Saccharideinheiten der Strukturen

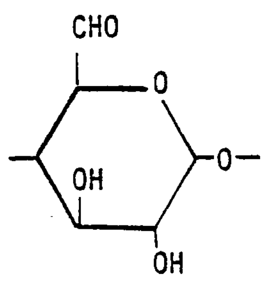

und

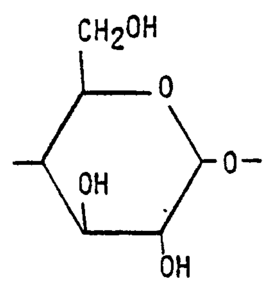

enthalten.

Als Saccharide werden im Sinne dieser Erfindung Mono-, Oligo- und Polysaccharide verstanden. Bei den Oligo- und Polysacchariden können 1,2-, 1,3-, 1,4- und 1,6-Verknüpfung der Monomereinheiten vorliegen, wobei sowohl Alpha- als auch Beta-Verknüpfung möglich ist. Die Saccharide können unverzweigt oder verzweigt sein. Vorzugsweise sind die Saccharide unverzweigt und haben einen Polymerisationsgrad von 1 bis 5. Die Saccharide können aus Hexosen aufgebaut sein, deren Glycoside primäre Hydroxylgruppen aufweisen. Die Hexosen können als Pyranosen oder Furanosen vorliegen. Geeignete Hexosen sind beispielsweise Glucose, Mannose, Galaktose oder Gulose. Vorzugsweise sind die Saccharide aus Glucose aufgebaut.

Das reduzierende Ende der Saccharide ist durch einen Alkyl- oder Acylrest substituiert.

Die Alkylgruppen können linear oder verzweigt sein. Sie können auch Doppelbindungen enthalten. Vorzugsweise sind sie jedoch linear und gesättigt und weisen 12 bis 16 C-Atome auf.

Auch die Acylgruppen können in der Kohlenwasserstoffkette linear oder verzweigt, gesättigt oder ungesättigt sein.

Bei dem Verfahren zur Herstellung der oxidierten substituierten Saccharide geht man von mit langkettigen Alkyl- oder Acylgruppen substituierten Sacchariden aus. Die Saccharide weisen bis zu 60 Saccharideinheiten auf, wobei 1 bis 5 Saccharideinheiten bevorzugt sind. Die Herstellung derartiger Produkte wird beispielsweise in DE-OS 36 19 796 beschrieben.

Die Alkylgruppen weisen 8 bis 22 C-Atome auf und können bis zu 2 Hydroxylgruppen enthalten. Vorzugsweise werden Alkylgruppen mit 12 bis 16 C-Atomen verwendet.

Die Acylgruppen sind aliphatisch und weisen 8 bis 22 C-Atome auf.

Diese substituierten Saccharide sind in Wasser unlöslich oder nur begrenzt löslich. Die Produkte werden einer Dreiphasenreaktion unterworfen: Ihre wäßrigen Mischungen werden an festen Katalysatoren mit gasförmigem Sauerstoff behandelt. Die Oxidation erfolgt durch Sauerstoff oder sauerstoffhaltige Gase.

Für die Oxidation können auch Gemische von substituierten Sacchariden eingesetzt werden. Vorzugsweise werden Alkylglucoside zu Alkylglucuronsäuren oxidiert.

Die Oxidation wird an Katalysatoren der VIII. Nebengruppe des Periodensystems durchgeführt.

Die Katalysatoren können auf geeigneten inerten Materialien wie Tonerde, Silikagel oder Aktivkohle niedergeschlagen sein. Vorzugsweise verwendet man Platin/Aktivkohle-Katalysatoren oder Platinoxid-Katalysatoren, von denen der Adams-Katalysator (J. Am. Chem. Soc. 46 (1924), 1683) bevorzugt wird.

Die Oxidation wird an 3 bis 30prozentigen wäßrigen Dispersionen der substituierten Saccharide bei 30 bis 100 °C durchgeführt, wobei vorzugsweise bei 40 bis 70 °C oxidiert wird.

Da bei der Oxidation Carboxylgruppen gebildet werden, fällt der pH-Wert der wäßrigen Mischung. Der pH-Wert wird bei der Reaktion durch Zugabe von Alkali im Bereich von 5 bis 11, vorzugsweise von 7 bis 9, gehalten. Bei Einstellung eines bestimmten pH-Wertes kann man den Fortgang der Reaktion am Alkaliverbrauch verfolgen. So ist es auch möglich, die Produkte definiert nur teilweise zu oxidieren. Es soll jedoch immer mindestens eine Carboxylgruppe pro Molekül gebildet werden.

Der Verlauf der Reaktion kann auch durch dünnschichtchromatographische oder gaschromatographische Analysen oder durch Probennahme, Anfärbung und photometrische Untersuchungen bestimmt werden.

Bei der Oxidation wird im allgemeinen so verfahren, daß man das substituierte Saccharid in Wasser dispergiert und den Katalysator, z. B. einen Platin/Aktivkohle-Katalysator mit 1 bis 10 % Platin, zugibt. Unter starkem Rühren wird Sauerstoff oder ein sauerstoffhaltiges Gas eingeleitet. Dabei kann es erforderlich sein, daß man die Schaumbildung durch einen Schaumbrecher reduziert. Durch Zugabe von Natronlauge oder Natriumhydrogencarbonat wird während der Reaktion der pH-Wert konstant gehalten. Nach beendeter Oxidation kann das Produkt durch Extraktion isoliert und durch Umkristallisation gereinigt werden.

Das erfindungsgemäße Verfahren ermöglicht die Herstellung von oxidierten substituierten Sacchariden mit langkettigen Alkyl- und Acylgruppen in hohen Ausbeuten von über 70 % bei geringem apparativen Aufwand.

Die durch das erfindungsgemäße Verfahren hergestellten oxidierten Saccharide mit langkettigen Alkyl- oder Acylgruppen zeichnen sich durch eine gut Wasserlöslichkeit aus. Sie sind biologisch leicht abbaubar und deshalb umweltfreundlich. Sie sind wegen ihrer hydrophilen und lipophilen Strukturelemente im Molekül für sich oder in Kombination mit anderen Tensiden (zu denen auch nichtoxidierte substituierte Saccharide gezählt werden können) für die Anwendung als Wasch- oder Reinigungsmittel gut geeignet.

Die folgenden Beispiele sollen die Erfindung verdeutlichen.

## Beispiel 1

In einem 1 l Vierhalskolben, der mit Gaseinleitungsrohr, Thermometer, pH-Elektrode, Tropftrichter und Rückflußkühler versehen ist, werden in eine Mischung aus 20 g Platin/Aktivkohlen-Katalysator, der 5 % Platin enthält, und 17 g Alpha-D-Dodecylglucosid und 500 ml Wasser 25 l/h Sauerstoff unter starkem Rühren eingeleitet.

Der pH-Wert wird durch Titrieren der entstandenen Säure mit 1 n wäßriger Natronlauge bei 9 konstant gehalten. Die Reaktionstemperatur beträgt 50 °C.

Es werden Proben gezogen, an denen nach Silylierung gaschromatographisch Ausbeuten bestimmt werden. Die Ausbeute an Alpha-D-Dodecylglucuronsäure beträgt nach 1 h 17 Mol-%, nach 2 h 39 Mol-%, nach 6 h 67 Mol-% und nach 12 h 77 Mol-%.

Nach beendeter Oxidation werden Ausgangsverbindungen durch Essigsäureethylester extrahiert. Dann wird durch Schwefelsäure angesäuert. Alpha-D-Dodecylglucuronsäure wird nun mit Essigsäureethylester extrahiert und durch Umkristallisieren aus Essigsäureethylester rein erhalten.

Die Struktur des Produkts wird durch $^1$H-NMR-Analyse in und $^{13}$C-NMR-Analyse bestätigt:

$^1$H-NMR: ([$D_4$]Methanol;ppm): Delta = 4.85 (d, H-1), 3.48 (dd, H-2), 3.55 (dd=t, H-3), 3.69 (dd=t, H-4), 4.09 (d, H-5), 3.76 (dt, $H_{Alkyl}$-1a), 3.53 (dt, H-1b), 1.67 (m, 2 H, $H_{Alkyl}$-2), 1.36 (bs, 18 H, $-(CH_2)-_{3-11}$), 0.95 (t, 3 H, $-CH_3$); $J_{1,2}$ = 3.7, $J_{2,3}$ = 9.6, $J_{3,4}$ = 9.4, $J_{4,5}$ = 9.8, $J_{Alkyl\ 1\ a,2}$ = 6.9, $J_{Alkyl\ 1\ b,\ 2}$ = 6.4, $J_{Alkyl\ 1a,\ 1\ b}$ = 9.7 Hz.

$^{13}$C-NMR: ([$D_6$]Aceton; ppm): Delta = 70.8 (t, $OCH_2$Alkyl); 73.3 (d, C-2 und C-4; 73.7 (d, C-3); 75.0 (d, C-5); 101 (d, C-1); 174,4 (S, C-6).

$(Alpha)_D^{20}$ = + 83.2 (c = 0.99 in Methanol)

Beispiel 2

In einem 5 l Vierhalskolben, der mit Gaseinleitungsrohr, Thermometer, pH-Elektrode, Tropftrichter und Rückflußkühler versehen ist, werden zu 3 l einer 5prozentigen wäßrigen Mischung von Alkylglucosiden mit linearen, gesättigten Alkylresten mit 12 bis 14 C-Atomen und einem mittleren Oligomerisationsgrad von 2,4 35 g Platin/Aktivkohle-Katalysator, der 10 % Platin enthält, gegeben.

Unter kräftigem Rühren werden 50 l/h Luft eingeleitet. Die Reaktionstemperatur beträgt 70 °C. Der pH-Wert wird bei 8 gehalten. Nach 7 h beträgt die gaschromatographisch bestimmte Ausbeute an oligomeren Alkylglucuronsäuren 80 %.

Die Isolierung der Produkte erfolgt wie in Beispiel 1.

Beispiel 3

Durch eine Mischung von 7 g Tetradecylglucosid und 0,3 g eines Adams Platinoxid-Katalysators in 200 ml Wasser werden bei 50 °C 10 l/h Luft geleitet. Dabei wird der Reaktionsansatz intensiv gerührt und durch portionsweise Zugabe von Natriumhydrogencarbonat auf einem pH-Wert von 8 gehalten.

Nach 10 h wird die Reaktion abgebrochen, der Katalysator abgetrennt und die entstandene Säure wie in Beispiel 1 durch Extraktion und Kristallisation aus Essigsäureethylester in 80 % Ausbeute in Form weißer Kristalle erhalten.

## Ansprüche

1. Verfahren zur Hersellung von oxidierten substituierten Sacchariden mit bis zu 60 Saccharideinheiten mit

- einem Alkylrest mit 8 bis 22 C-Atomen, der bis zu 2 Hydroxylgruppen enthalten kann, oder einem aliphatischen Acylrest mit 8 bis 22 C-Atomen und
- mindestens einer Saccharideinheit der Struktur

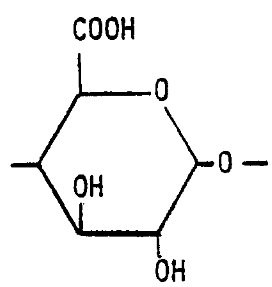

dadurch gekennzeichnet,

daß man Saccharide mit einem Alkylrest mit 8 bis 22 C-Atomen, der bis zu 2 Hydroxylgruppen enthalten kann, oder mit einem aliphatischen Acylrest mit 8 bis 22 C-Atomen in einer wäßrigen Mischung durch Sauerstoff oder sauerstoffhaltige Gase an einem Katalysator der VIII. Nebengruppe des Peroxidsystems oxidiert.

2. Verfahren nach Anspruch 1,
dadurch gekennzeichnet,
daß man an einem Platin/Aktivkohle-Katalysator oder an einem Platinoxid-Katalysator oxidiert.

3. Verfahren nach Anspruch 1,
dadurch gekennzeichnet,
daß man unverzweigte Saccharide mit einem Polymerisationsgrad von 1 bis 5 und einem Alkylrest mit 8 bis 22 C-Atomen oxidiert.

4. Verfahren nach Anspruch 3,
dadurch gekennzeichnet,
daß man 3 bis 30prozentige wäßrige Mischungen von Alkylsacchariden einsetzt und die Oxidation bei 30 bis 100 °C durchführt.

5. Verfahren nach Anspruch 4,
dadurch gekennzeichnet,
daß man Alkylglucoside mit Alkylgruppen mit 12 bis 16 C-Atomen bei 40 bis 70 °C und einem pH-Wert von 5 bis 11, vorzugsweise von 7 bis 9, zu Alkylglucuronsäuren oxidiert.